# EUROPÄISCHE PATENTANMELDUNG

(11) **EP 0 572 909 A1**
(43) Veröffentlichungstag der Anmeldung: **08.12.1993**
(21) Anmeldenummer: 93108471.9
(22) Anmeldetag: 26.05.1993
(51) Int. Cl.: C07D 233/54, C07D 213/56, A61K 31/415, A61K 31/44, C07C 317/46

(54) **Aminosäurederivate as Renininhibitoren**

(30) Priorität: 04.06.1992 CH 1795/92
(71) Anmelder: F. HOFFMANN-LA ROCHE AG, CH-4002 Basel (CH)
(72) Erfinder: Branca, Quirico, CH-4102 Binningen (CH); Stadler, Heinz, CH-4310 Rheinfelden (CH); Vieira, Eric, CH-4053 Basel (CH); Wostl, Wolfgang, W-7889 Grenzach (DE)
(74) Vertreter: Kellenberger, Marcus, Dr.

(57) **Zusammenfassung**

Die Verbindungen der Formel 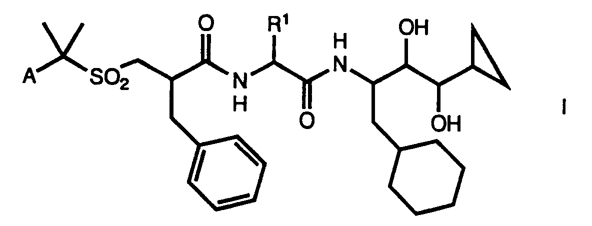
worin R¹ Imidazolylmethyl oder Pyridylmethyl und A Carboxyl, Benzyloxycarbonyl, Hydroxymethyl oder Alkylcarbonyloxymethyl bedeuten,
in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können nach verschiedenen, an sich Bekannten Verfahren hergestellt werden.

## Beschreibung

Die vorliegende Erfindung betrifft Aminosäurederivate. Im speziellen betrifft sie Aminosäurederivate der allgemeinen Formel 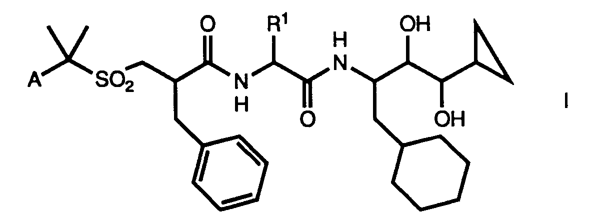
worin R¹ Imidazolylmethyl oder Pyridylmethyl und A Carboxyl, Benzyloxycarbonyl, Hydroxymethyl oder Alkylcarbonyloxymethyl bedeuten,
in Form von optisch reinen Diastereomeren, Diastereomerengemische, diastereomeren Racematen oder Gemischen von diastereomeren Racematen sowie pharmazeutisch verwendbare Salze dieser Verbindungen.

Diese Verbindungen zeichnen sich durch wertvolle pharmakodynamische Eigenschaften aus und können zur Bekämpfung oder Verhütung von Krankheiten verwendet werden. Sie besitzen insbesondere eine Renin-hemmende Wirkung und eignen sich denmach zur Behandlung von Bluthochdruck und Herzinsuffizienz.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Die Verbindungen der obigen Formel I sind generisch bereits aus beispielsweise EP-A-0.309.766 und EP-A-0.377.139 bekannt, doch wurde überraschenderweise gefunden, dass sie sich durch eine ausgeprägte orale Aktivität auszeichnen. Von den in den beiden europäischen Patentpublikationen spezifisch genannten Verbindungen sind nur deren zwei als oral wirksam beschrieben, nämlich das Endprodukt von Beispiel 16 von EP-A-0.309.766 bzw. das Endprodukt von Beispiel 1 von EP-A-0.377.139. Ein Vergleich der oralen Wirkung der erfindungsgemässen Verbindungen mit der besser wirksamen vorbeschriebenen Verbindung (Beispiel 1 von EP-A-0.377.139) zeigt eine eindeutig höhere orale Aktivität für die Verbindungen der obigen Formel I, wie den weiter unten diskutierten Abbildungen 1-3 ohne weiteres entnommen werden kann.

Der in der vorliegenden Beschreibung im Ausdruck "Alkylcarbonyloxymethyl" bzw. "Alkylcarbonyl" aufscheinende Alkylrest betrifft geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, tert-Butyl, Pentyl, Hexyl und dergleichen.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen, oder, für den Fall, dass A Carboxyl bedeutet, auch mit anorganischen oder organischen Basen, wie Natrium- oder Kaliumhydroxyd, Ammoniak, Triäthylamin, Diisopropyläthylamin, Pyridin und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens vier asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugt sind solche Verbindungen der Formel I, worin A Hydroxymethyl oder Alkylcarbonyloxymethyl, besonders bevorzugt Alkylcarbonyloxymethyl, insbesondere C₁₋₄-Alkylcarbonyloxymethyl, bedeutet Falls A keine der oben genannten Bedeutungen besitzt, so sind diejenigen Verbindungen der Formel I bevorzugt, worin A Carboxyl bedeutet.

Aus dem obigen folgt, dass solche Verbindungen der Formel I besonders bevorzugt sind, worin A C₁₋₄-Alkylcarbonyloxymethyl bedeutet.

Ganz speziell bevorzugte Verbindungen der Formel I sind:
2,2-Dimethylpropionsäure 2-[(S)- oder 2-[(R)-2-[(S)-1 [(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester,
2,2-Dimethylpropionsäure 2-[(R)- oder 2-[(S)-2-[(S)-1-[(1S,2R,3S)-1-cyclohexylmethyl-3-cyclopropyl-2,3-dihydroxypropylcarbamoyl]-2-pyridin-3-yläthylcarbamoyl]-3-phenylpropylsulfonyl]-2-methylpropylester und
2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-pyridin-3-yläthylcarbamoyl]-3-phenylpropylsulfonyl]-2-methylpropylester.

Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man
a) eine Verbindung der allgemeinen Formel 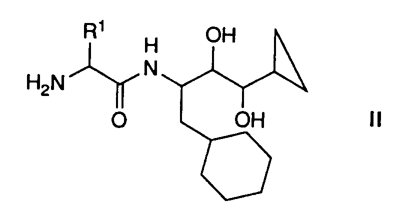 worin R¹ die oben angegebene Bedeutung besitzt, mit einer Säure der allgemeinen Formel 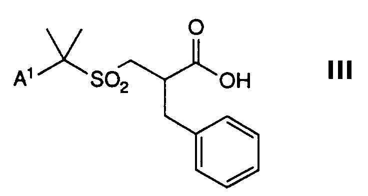 worin A¹ Benzyloxycarbonyl, Hydroxymethyl oder Alkylcarbonyloxymethyl bedeutet,
   oder einem aktivierten Derivat davon umsetzt, oder
b) die Verbindung der Formel 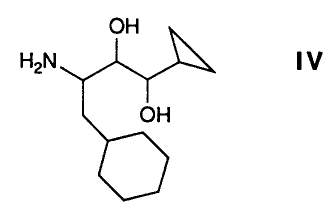 mit einer Säure der allgemeinen Formel 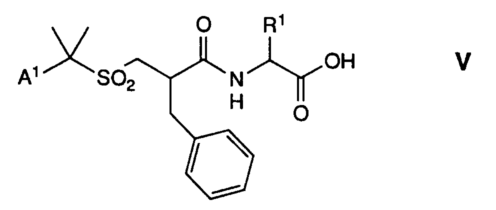 worin A¹ und R¹ die oben angegebene Bedeutung besitzen, oder einem aktivierten Derivat davon umsetzt, oder
c) zur Herstellung einer Verbindung der Formel I, worin A Carboxyl bedeutet, in einer Verbindung der Formel I, worin A Benzyloxycarbonyl bedeutet, die Benzylgruppe abspaltet, und
d) erwünschtenfalls ein Gemisch diastereomerer Racemate in die diastereomeren Racemate oder optisch reinen Diastereomeren auftrennt, und/oder
e) erwünschtenfalls ein Diastereomerengemisch in die optisch reinen Diastereomeren auftrennt, und/oder
f) erwünschtenfalls eine erhaltene Verbindung in ein pharmazeutisch verwendbares Salz überführt.

Die Acylierung einer Verbindung der Formel II mit einer Säure der Formel III oder einem aktivierten Derivat davon erfolgt nach an sich in der Peptid-Chemie bekannten Methoden. Geeignete Acylierungsmittel sind insbesondere aktivierte Derivate, wie Ester, gemischte Ester, Säurehalogenide, Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0°C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Äther, wie Diäthyläther, Tetrahydrofuran oder Dioxan, und dergleichen in Frage. Weiterhin kann die Acylierung in Gegenwart eines Kondensationsmittels, wie HBTU [O-Benzotriazolyl-N,N,N',N'-tetramethyluronium-hexafluorophosphat], BOP [Berzotriazol-1-yloxy-bis(dimethylamino)phosphonium-hexafluorophosphat], BOPC [Bis(2-oxo-2-oxazolidinyl)phosphinchlorid], HOBT [N-Hydroxybenzotriazol], DCC[Dicyclohexylcarbodiimid-hydrochlorid], EDC [N-(3-Dimethylaminopropyl)-N'-äthylcarbodiimid-hydrochlorid] und dergleichen, erfolgen. Die Reaktion wird zweckmäßigerweise in einem unter den Reaktionsbedingungen inerten organischen Lösungsmittel oder Lösungsmittelgemisch bei einer Temperatur zwischen etwa 0° und 50°C, vorzugsweise bei etwa Raumtemperatur, durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel IV mit einer Säure der Formel V bzw. einem aktivierten Derivat davon erfolgt ebenfalls nach an sich in der Peptid-Chemie bekannten Methoden, d.h. unter den gleichen Bedingungen wie oben für die Acylierung einer Verbindung der Formel II angegeben wurde. Beispiele geeigneter aktivierter Verbindungen der Formel V sind ebenfalls Säurehalogenide, Säureanhydride, gemischte Anhydride, Ester, gemischte Ester, und dergleichen.

Die Abspaltung der Benzylgruppe gemäß Verfahrensvariante c) erfolgt ebenfalls nach an sich bekannten Methoden, zweckmässig hydrogenolytisch.

Die Ausgangsstoffe der Formel II sind teilweise neu und teilweise bekannt. Diese Verbindungen können hergestellt werden, indem man die Verbindung der Formel IV mit einer Verbindung der allgemeinen Formel 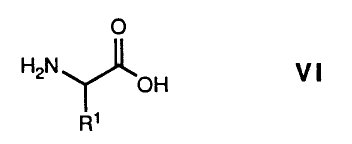
worin R¹ die oben angegebene Bedeutung besitzt,
umsetzt. Diese Umsetzung erfolgt nach in der Peptid-Chemie bekannten Methoden, d.h. unter den Reaktionsbedingungen, wie sie weiter oben für die Acylierung einer Verbindung der Formel II beschrieben sind.

Der Ausgangsstoff der Formel IV, d. h. das 3-Amino-4-cyclohexyl-1-cyclopropyl-1,2-butandiol, ist bekannt.

Die Verbindungen der Formel VI sind bekannt oder können in Analogie zur Herstellung der bekannten Verbindungen erhalten werden.

Die Säuren der Formel III und ihre aktivierten Derivate sind neu und ebenfalls Gegenstand vorliegender Erfindung. Im nachstehenden Reaktionsschema ist die Herstellung der Säuren der Formel III formelmäßig dargestellt. Die aktivierten Derivate können im Hinblick auf den Stand der Technik und unter Berücksichtigung der Natur des aktivierten Derivates durch jeden Fachmann ohne weiteres hergestellt werden.

Die Säuren der Formel V und ihre aktivierten Derivate sind ebenfalls neu und Gegenstand vorliegender Erfindung. Sie können leicht hergestellt werden, indem man eine Säure der Formel III mit einer Verbindung der Formel VI umsetzt. Die Umsetzung erfolgt nach in der Peptid-Chemie bekannten Methoden, d.h. unter den Reaktionsbedingungen, wie sie weiter oben für die Acylierung einer Verbindung der Formel II beschrieben sind. Die aktivierten Derivate der Säuren der Formel V können wie diejenigen der Säuren der Formel III durch jeden Fachmann ohne weiteres hergestellt werden.

Bei den im Reaktionsschema dargestellten Schritte handelt es sich durchwegs um in der synthetischen Chemie übliche Reaktionen, die alle nach an sich bekannten Methoden durchgeführt werden. Bezüglich der genauen Reaktionsbedingungen für die im Reaktionsschema dargestellten Schritte wird auf den Beispielteil verwiesen. Im Reaktionsschema bedeuten die Symbole R² Aralkyl, insbesondere Benzyl, oder Alkyl, insbesondere t-Butyl, R²¹ Alkyl, insbesondere t-Butyl, R³ Benzyl und Z Alkylcarbonyl.

Die im Reaktionsschema verwendeten Ausgangsstoffe der Formeln VII und VIII sind bekannt. 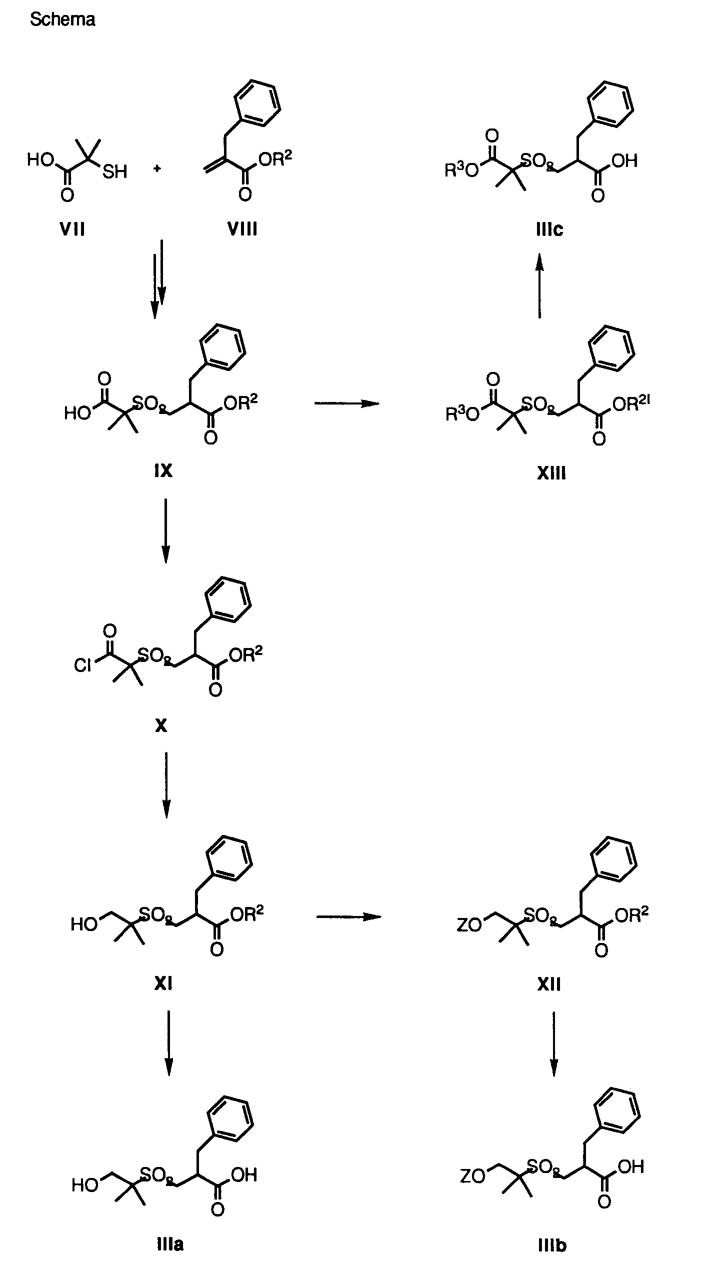
Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die orale Wirkung von Renin-Hemmern kann experimentell mittels des nachstehend beschriebenen In vivo-Tests gezeigt werden:

### Blutdrucksenkende Wirkung im Affenmodell

Der blutdrucksenkende Effekt der Verbindungen wurde in normotensiven Totenkopfäffchen beiderlei Geschlechts (Gewicht 400-700 g) gemessen. Die Natrium-Depletion wurde durch subkutane Injektion von jeweils 5 mg/kg Furosemid 66, 42 und 18 Stunden vor dem Experiment erreicht.

Die Verbindungen wurden oral als Methansulfonsäuresalze in wässriger Lösung in einer Dosis von 3 mg/kg verabreicht. Der arterielle Blutdruck wurde nach dem in Hypertension 1991; 18: 22-31 publizierten Verfahren gemessen.

Die in diesem Test erhaltenen Resultate sind in den folgenden Abbildungen 1-3 zusammengefasst. 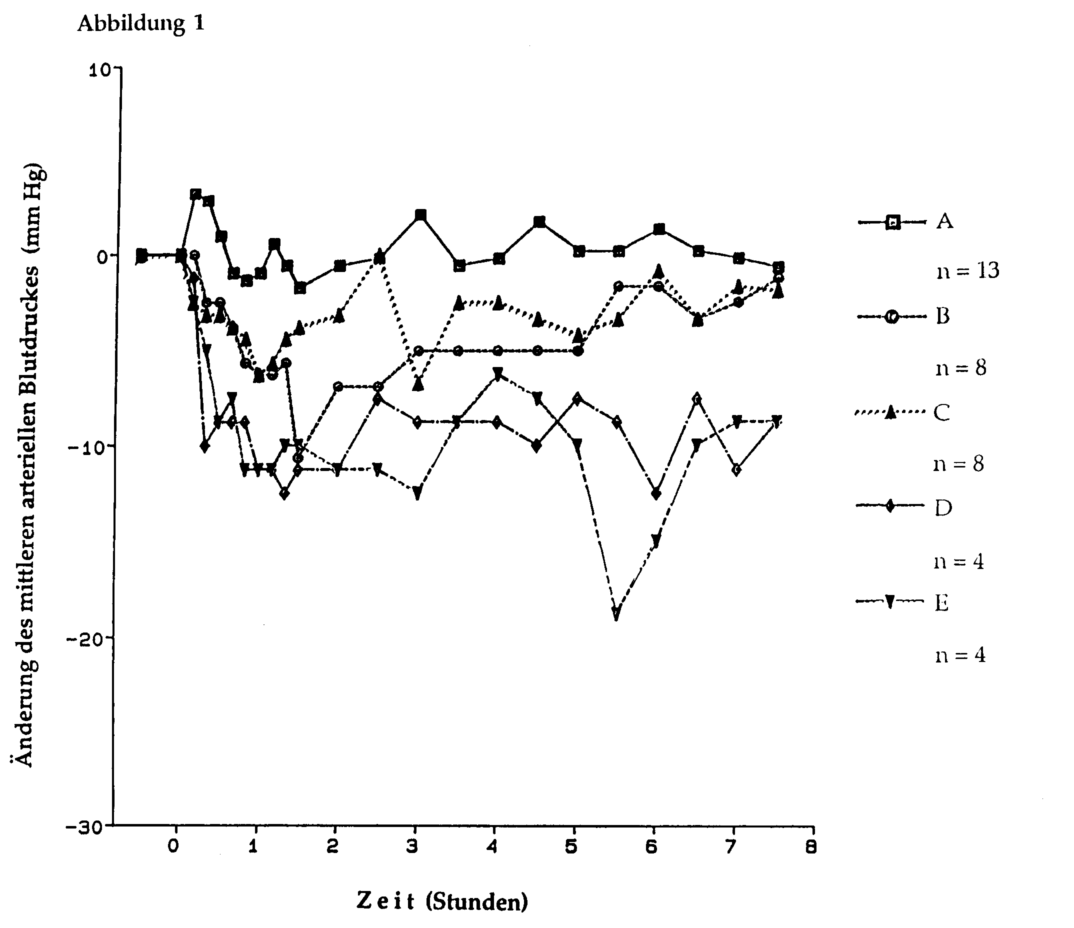 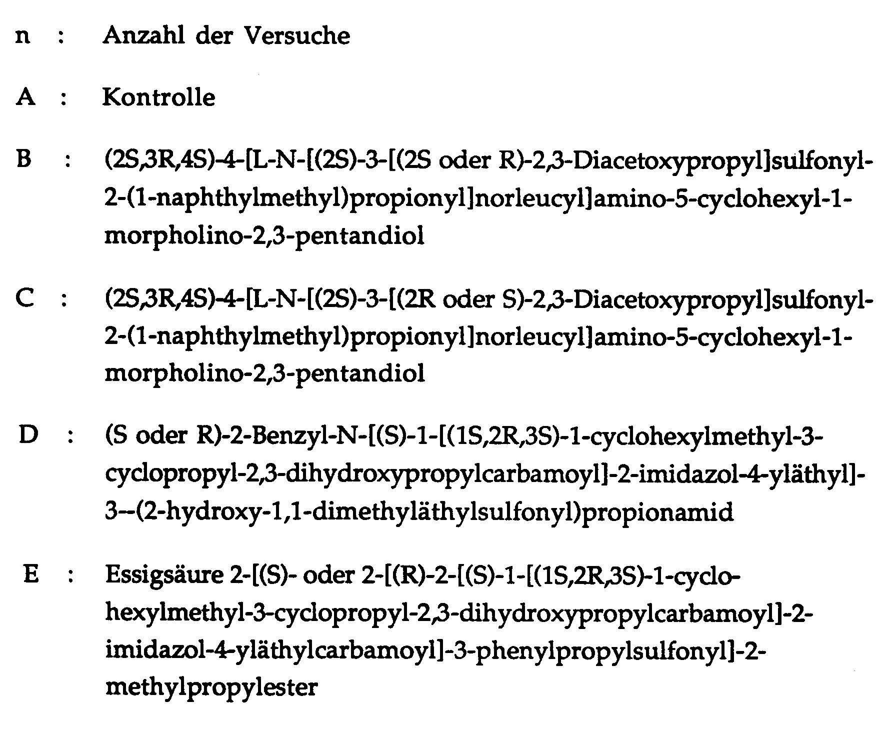 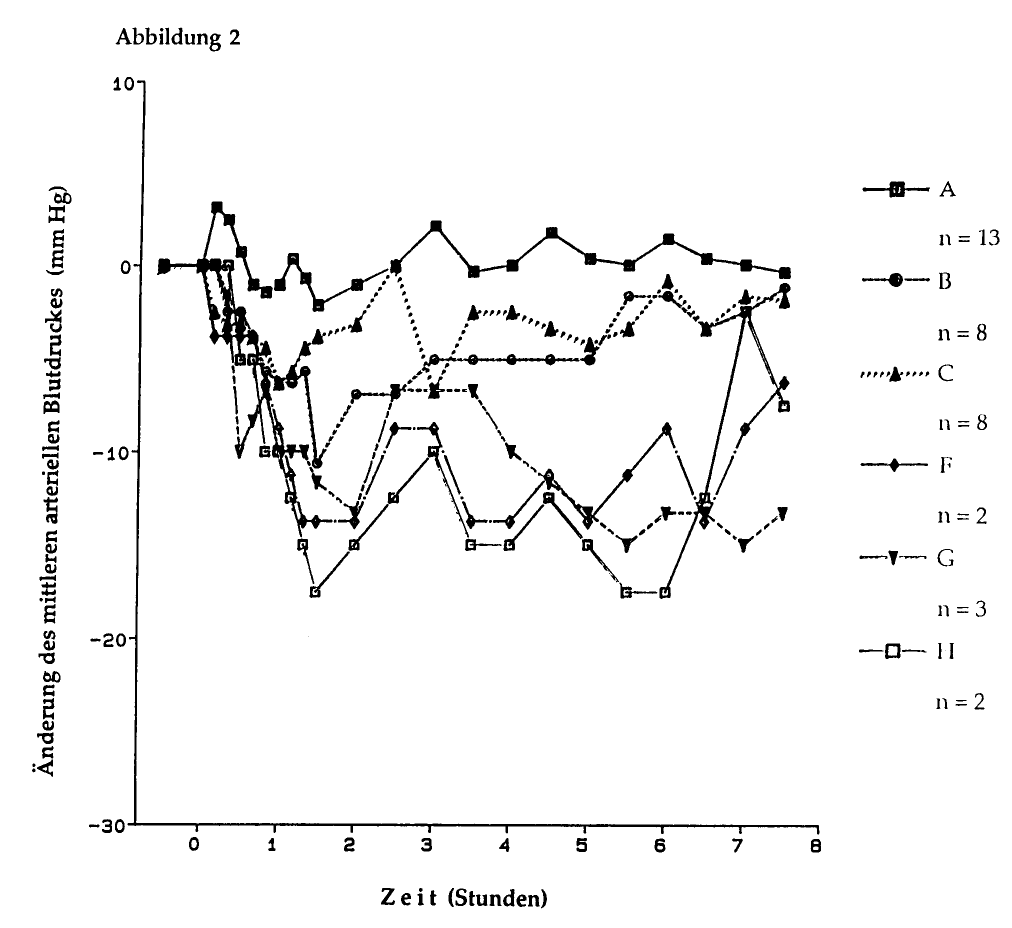 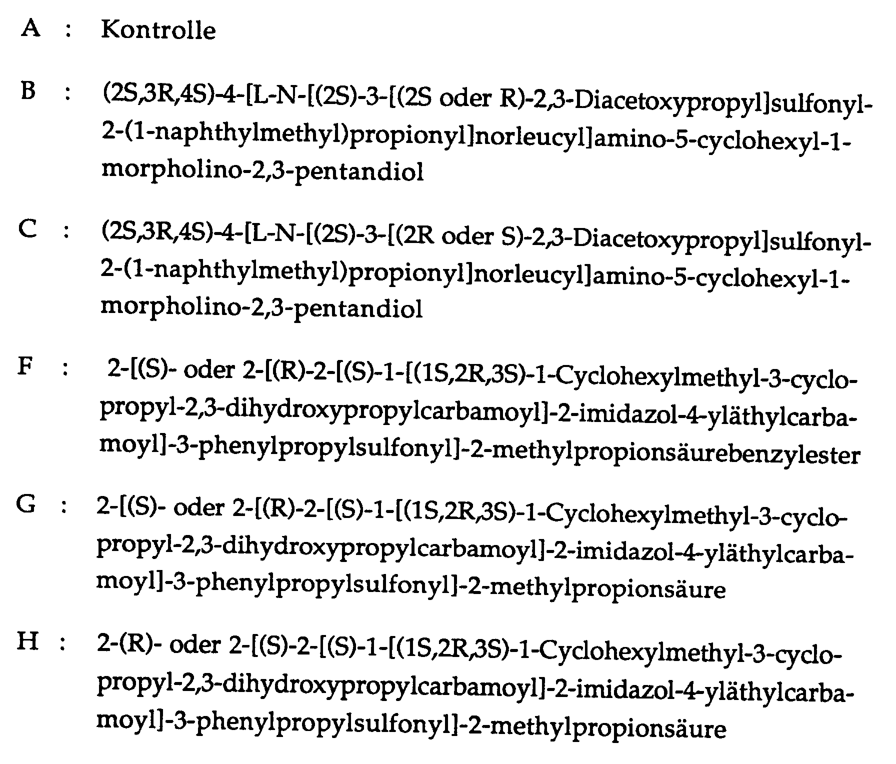 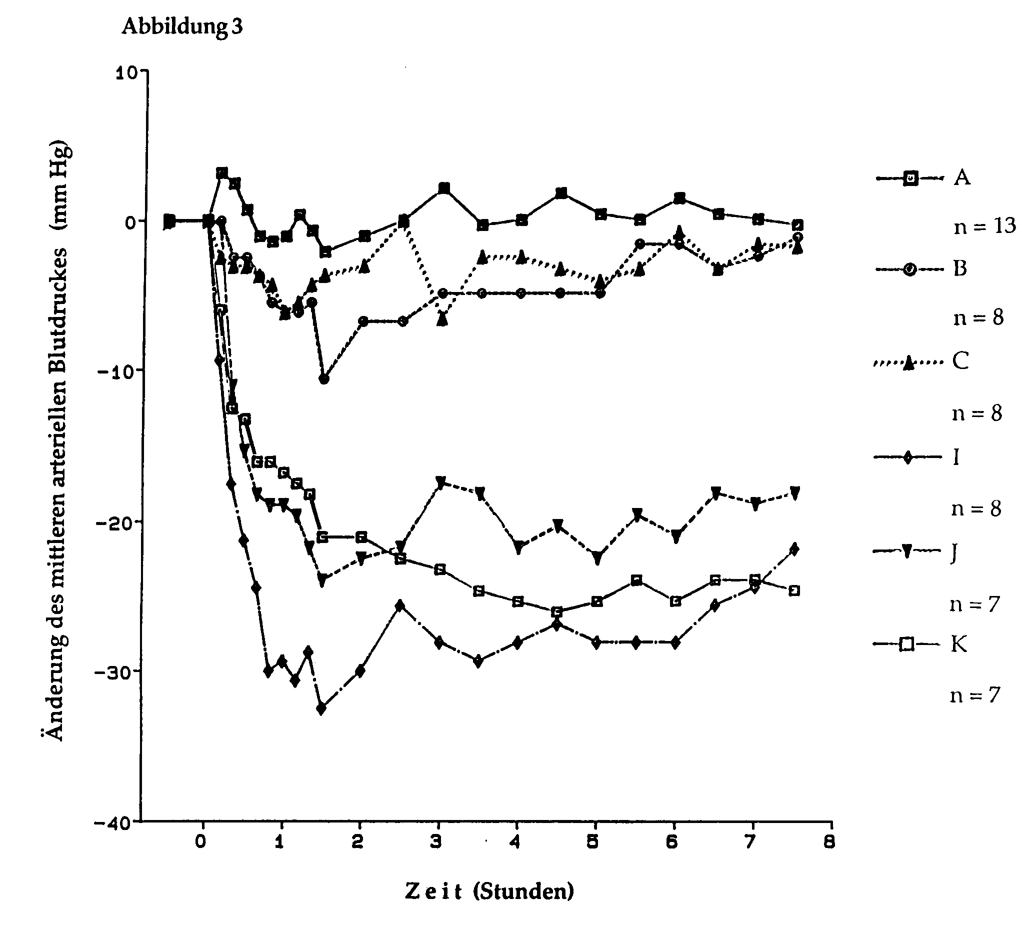 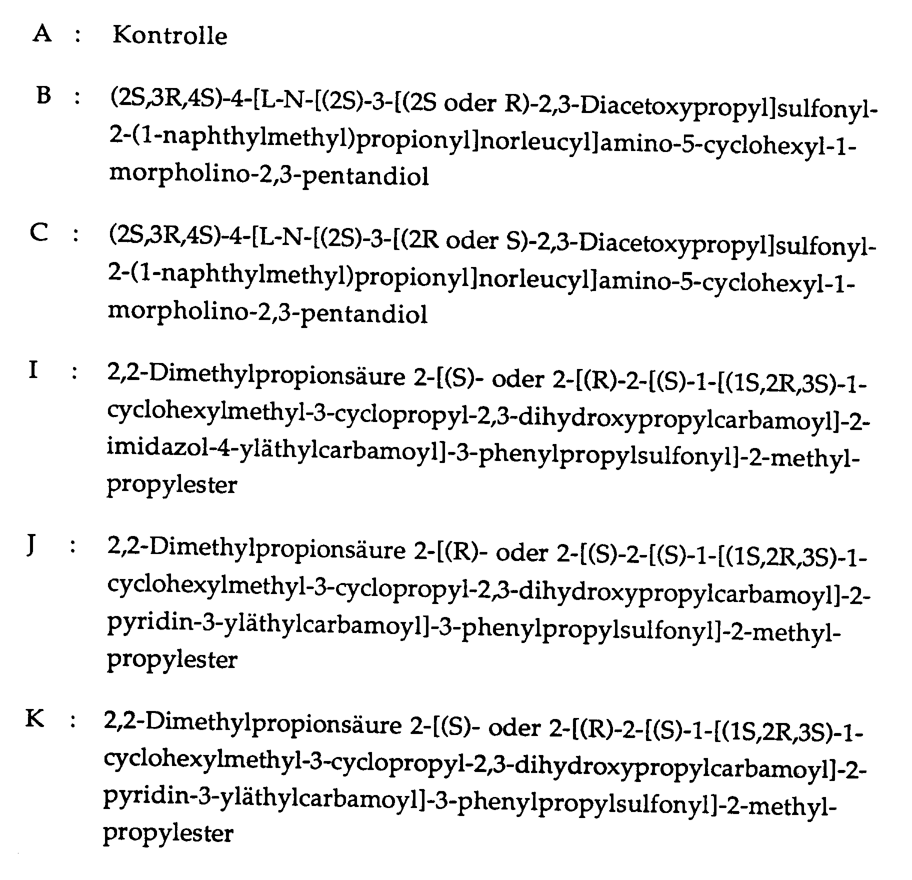
Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser. Polyole, Sacharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen verwendet:
- His-OH: = L-Histidin
- Fmoc: = 9-Fluorenylmethoxycarbonyl
- DBU: = 1,8-Diazabicyclo[5.4.0]undec-7-en[1,5-5]

### Beispiel 1

Ein Gemisch von 2.33 g (6.06 mMol) (RS)-2-Benzyl-3-[2-(2,2-dimethylpropionyloxy)-1,1-dimethyläthylsulfonyl]propionsäure, 2.0 g (5.49 mMol), (S)-2-Amino-N-[(1S,2R,3S)-1-cyclohexylmethyl-3-cyclopropyl-2,3-dihydroxypropyl]-3-imidazol-4-ylpropionamid, 0.61 g (6.06 mMol) Triäthylamin, 0.95 g (6.06 mMol) HOBT und 2.3 g (6.06 mMol) HBTU in 50 ml Dimethylformamid wurde über Nacht bei Raumtemperatur gerührt. Danach wurde das Reaktionsgemisch im Hochvakuum eingedampft. Der Rückstand wurde in 500 ml Essigester aufgenommen und zweimal mit je 200 ml gesättigter Natriumhydrogencarbonatlösung gewaschen. Die Essigesterphase wurde über Natriumsulfat getrocknet und anschließend unter vermindertem Druck eingedampft. Der Rückstand (4.98 g) wurde zur Reinigung und Auftrennung der beiden epimeren Produkte zweimal an 400 g Kieselgel unter Verwendung eines 97:3:0.1-Gemisches von Methylenchlorid, Methanol und Pyridin als Eluierungsmittel chromatographiert. Der weniger polare 2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester wurde aus Essigester/Hexan ausgefällt und ergab 1.3 g eines farblosen Festkörpers, MS : 731 (M+H)⁺. Der polarere 2,2-Dimethylpropionsäure 2-[(R)- oder 2-[(S)-2-[(S)1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester ergab nach dem Lyophilisieren aus Dioxan/Wasser 1.08 g eines farblosen Pulvers, MS : 731 (M+H)⁺.

Das als Ausgangsstoff eingesetzte 2-Amino-N-[(1S,2R,3S)-1-cyclohexylmethyl-3-cyclopropyl-2,3-dihydroxypropyl]-3-imidazol-4-ylpropionamid wurde wie folgt hergestellt:
(a) (4S,5R)-4-Cyclohexylmethyl-5-[(S)-cyclopropylhydroxymethyl]-2,2-dimethyl-3-oxazolidincarbonsäure-tert-butylester und (4S,5R)-4-Cyclohexylmethyl-5-[(R)-cyclopropylhydroxymethyl]-2,2-dimethyl-3-oxazolidonecarbonsäure-tert-butylester :
   Zu einer Lösung der aus 3.94 ml (49 mMol) Bromcyclopropan und 1.2 g (0.049 Grammatom) Magnesiumspänen in 22 ml Tetrahydrofuran hergestellten Grignardverbindung wurde bei ca. 15° eine Lösung von 3.21 g (4S,5R)-4-cyclohexylmethyl)-5-formyl-2,2-dimethyl-3-oxazolidincarbonsäure-tert-butylester [WO 87/05302] in 25 ml Tetrahydrofuran getropft, und das Reaktionsgemisch anschließend während 16 Stunden bei Raumtemperatur unter Argon gerührt. Danach goß man das Reaktionsgemisch auf 40 ml einer eiskalten gesättigten Ammoniumchloridlösung und extrahierte zweimal mit je 50 ml Essigester. Die Essigesterextrakte wurden mit 40 ml eiskalter gesättigter Ammoniumchloridlösung gewaschen, dann vereinigt, über Natriumsulfat getrocknet und unter vermindertem Druck eingedampft. Zur Reinigung wurde der Rückstand (4.33 g) über eine mit Toluol und 1% Triäthylamin aufgezogene Säule von 110 g Kieselgel mit einem 95:5-Gemisch von Toluol und Essigester als Eluierungsmittel chromatographiert. Man erhielt 1.9 g (4S,5R)-4-Cyclohexylmethyl-5-[(R)-cyclopropylhydroxymethyl]-2,2-dimethyl-3-oxazolidincarbonsäure-tert-butylester , MS : 368 (M+H)⁺, und 0.5 g (4S,5R)-4-Cyclohexylmethyl-5-[(S)-cyclopropylhydroxymethyl]-2,2-dimethyl-3-oxazolidincarbonsäure-tert-butylester , MS : 368 (M+H)⁺, jeweils als farbloses Öl.
(b) (1S,2R,3S)-3-Amino-4-cyclohexyl-1-cyclopropylbutan-1,2-diol:
   1.42 g (3.86 mMol) (4S,5R)-4-Cyclohexylmethyl-5-[(R)-cyclopropylhydroxymethyl]-2,2-dimethyl-3-oxazolidincarbonsäure-tert-butylester , gelöst in 15 ml Methanol und 10 ml Wasser, wurden mit 4 ml 7.5 N Salzsäure versetzt und 3 Stunden bei 50° gerührt. Die Reaktionslösung wurde im Eisbad auf 3° abgekühlt, mit 4 ml 7.5 N Natronlauge tropfenweise versetzt und 1 Stunde nachgerührt. Die erhaltene Suspension wurde unter vermindertem Druck eingedampft, zweimal mit 10 ml Toluol azeotrop entwässert, und der Rückstand dreimal mit 10 mi eines 95:5-Gemisches von Methylenchlorid und Methanol ausgerührt. Der unlösliche Rückstand wurde abfiltriert, und das Filtrat unter vermindertem Druck eingedampft. Das erhaltene Rohprodukt (1.14 g) wurde in 15 ml Äther suspendiert und danach abgesaugt. Man erhielt 0.58 g (1S,2R,3S)-3-Amino-4-cyclohexyl-1-cyclopropylbutan-1,2-diol als farblose Kristalle, Smp. 141-142°.
(c) (S)-2-Amino-N-[(1S,2R,3S)-1-cyclohexylmethyl-3-cyclopropyl-2,3-dihydroxypropyl]-3-imidazol-4-ylpropionamid :
   Ein Gemisch von 343 mg (1.51 mMol) (1S,2R,3S)-3-Amino-4-cyclohexyl-1-cyclopropylbutan-1,2-diol, 995 mg (1.66 mMol) (Fmoc)₂His-OH, 0.21 ml (1.61 mMol) 4-Äthylmorpholin, 449 mg (3.22 mMol) HOBT und 347 mg (1.81 mMol) EDC in 20 ml Dimethylformamid wurde über Nacht bei Raumtemperatur stehengelassen. Danach wurde das Reaktionsgemisch im Hochvakuum eingedampft, der Rückstand auf ein Gemisch von Eis und 90 ml Natriumbicarbonatlösung gegossen und dreimal mit je 150 ml Essigester extrahiert. Die drei Essigesterextrakte wurden nacheinander mit 70 ml gesättigter Ammoniumchloridlösung, 70 ml 2N Natriumbicarbonatlösung und 70 ml gesättigter Natriumchloridlösung gewaschen, vereinigt, über Magnesiumsulfat getrocknet, filtriert und eingedampft. Das erhaltene Rohprodukt wurde in 60 ml Methylenchlorid und 2 ml Piperidin 3 Stunden bei Raumtemperatur gerührt. Danach wurde das Reaktionsgemisch eingedampft und der Rückstand aus 50 ml Hexan trituriert und abfiltriert. Das Filtrat wurde mit einem 65:10:1-Gemisch von Methylenchlorid, Methanol und Ammoniak als Eluierungsmittel an 70 g Kieselgel chromatographiert, wobei man 390 mg (S)-2-Amino-N-[(1S,2R,3S)-1-cyclohexylmethyl-3-cyclopropyl-2,3-dihydroxypropyl]-3-imidazol-4-ylpropionamid als farblosen Schaum erhielt; MS : 365 (M+H)⁺.
   Die als Ausgangsstoff eingesetzte (RS)-2-Benzyl-3-[2-(2,2-dimethylpropionyloxy)-1,1-dimethyläthylsulfonyl]propionsäure wurde wie folgt hergestellt:
(d) (RS)-2-(2-Benzyloxycarbonyl-3-phenylpropylsulfonyl)-2-methylpropionsäure:
   Zu einer Lösung von 3.3 g (27.7 mMol) 2-Mercaptoisobuttersäure [Can.J. Chem. 61(8), 1872] und 6.9 g (27.7 mMol) 2-Benzylacrylsäurebenzylester [EP-A 0.117.429] wurden 8.4 g (55.4 mMol) DBU getropft, wobei die Temperatur des Reaktionsgemisches zwischen 5 und 10° gehalten wurde. Nach beendeter Zugabe wurde weitere 5 Stunden bei 10° gerührt. Anschließend wurde das Reaktionsgemisch unter Eiskühlung mit 22.5 g (36.6 mMol) Kaliummonopersulfat-tripelsalz, suspendiert in 450 ml Wasser, tropfenweise versetzt, wobei die Temperatur unterhalb 10° gehalten wurde. Danach wurde eine Stunde bei der gleichen Temperatur weitergerührt, dann auf 0° abgekühlt und nochmals 22.5 g (36.6 mMol) Kaliummonopersulfat-tripelsalz spatelweise zugegeben. Man ließ langsam auf Raumtemperatur erwärmen und rührte 15 Stunden weiter. Zur Aufarbeitung wurde mit 200 ml Wasser verdünnt und viermal mit je 60 ml Essigester extrahiert. Die vereinigten organischen Extrakte wurden über Natriumsulfat getrocknet und unter vermindertem Druck eingedampft bis das Produkt zu kristallisieren begann. Dann wurden unter Rühren 30 ml Hexan und 20 ml Äther zugefügt, und anschließend das ausgefallene Produkt abgesaugt und getrocknet. Es wurden 9.7 g (RS)-2-(2-Benzyloxycarbonyl-3-phenylpropylsulfonyl)-2-methylpropionsäure als farbloser Festkörper erhalten; MS : 422 (M+NH₄)⁺.
(e) (RS)-2-(2-Benzyloxycarbonyl-3-phenylpropylsulfonyl)-2-methylpropionsäurechlorid:
   Eine Lösung von 9.0 g (22.2 mMol) (RS)-2-(2-Benzyloxycarbonyl-3-phenylpropylsulfonyl)-2-methylpropionsäure und 9.6 ml (112.3 mMol) Oxalylchlorid in 18 ml Tetrahydrofuran wurde 18 Stunden auf 50° erwärmt. Danach wurde die Reaktionslösung unter vermindertem Druck eingedampft. Der Rückstand wurde zweimal mit jeweils 250 ml Toluol aufgenommen und unter vermindertem Druck eingedampft. Das (RS)-2-(2-Benzyloxycarbonyl-3-phenylpropylsulfonyl)-2-methylpropionsäurechlorid wurde als gelbliches Öl in quantitativer Ausbeute erhalten und ohne Reinigung und Charakterisierung in die folgende Stufe eingesetzt.
(f) (RS)-2-Benzyl-3-(2-hydroxy-1,1-dimethyl-äthylsulfonyl)propionsäurebenzylester :
   Eine Lösung von 9.4 g (22.2 mMol) (RS)-2-(2-Benzyloxycarbonyl-3-phenylpropylsulfonyl)-2-methylpropionsäurechlorid in 45 ml Tetrahydrofuran wurde bei 0° innerhalb von 15 Minuten tropfenweise mit 2.3 ml (23.3 mMol) 10M Boran-Dimethylsulfidkomplex versetzt und anschließend bei Raumtemperatur 3 Stunden nachgerührt. Danach wurde auf 0° abgekühlt und in der Folge ca. 2 ml Methanol, 30 ml Wasser und 30 ml gesättigte Natriumhydrogencarbonatlösung zugegeben. Das Gemisch wurde dann dreimal mit je 35 ml Äther extrahiert, und anschliessend die vereinigten organischen Phasen über Natriumsulfat getrocknet und unter vermindertem Druck eingedampft. Das Rohprodukt wurde in 50 ml Äther digeriert, wobei nach dem Absaugen und Trocknen 6.7 g (RS)-2-Benzyl-3-(2-hydroxy-1,1-dimethyläthylsulfonyl)propionsäurebenzylester als farbloser Festkörper erhalten wurden; MS : 408 (M+NH₄)⁺.
(g) (RS)-2-Benzyl-3-[2-(2,2-dimethylpropionyloxy)-1,1-dimethyläthylsulfonyl]propionsäurebenzylester :
   Zu einer Lösung von 2.0 g (5.12 mMol) (RS)-2-Benzyl-3-(2-hydroxy-1,1-dimethyläthylsulfonyl)propionsäurebenzylester und 60 mg (0.5 mMol) N,N'-Dimethylaminopyridin in 20 ml trockenem Pyridin wurden bei Raumtemperatur 0.74 g (6.14 mMol, 1.2 Moläq) Pivaloylchlorid gegeben. Man erwärmte das Reaktionsgemisch auf 40° und rührte 6 Stunden bei dieser Temperatur weiter. Zur Aufarbeitung wurde die abgekühlte Reaktionslösung im Wasserstrahlvakuurn eingedampft. Der Rückstand wurde zur Reinigung an Kieselgel unter Verwendung eines 99:1-Gemisches von Methylenchlorid und Methanol als Eluierungsmittel chromatographiert. Es wurden 2.18 g (90 % d.Th.) (RS)-2-Benzyl-3-[2-(2,2-dimethylpropionyloxy)-1,1-dimethyläthylsulfonyl]propionsäurebenzylester als farblose Kristalle erhalten; MS : 475 (M+H)⁺.
(h) (RS)-2-Benzyl-3-[2-(2,2-dimethylpropionyloxy)-1,1-dimethyläthylsulfonyl]propionsäure :
   Eine Lösung von 2.17 g (4.57 mMol) (RS)-2-Benzyl-3-[2-(2,2-dimethylpropionyloxy)-1,1-dimethyläthylsulfonyl]propionsäurebenzylester in 100 ml Methanol wurde mit 0.5 g Palladium/Kohle (5 %ig) versetzt und bei Raumtemperatur unter Normaldruck 90 Minuten hydriert. Zur Aufarbeitung wurde der Katalysator abfiltriert und dreimal mit je 30 ml Methanol nachgewaschen. Nach dem Eindampfen im Wasserstrahlvakuum wurden 1.55 g (88 % d.Th.) (RS)-2-Benzyl-3-[2-(2,2-dimethylpropionyloxy)-1,1-dimethyläthylsulfonyl]propionsäure als farblose Kristalle erhalten; MS : 385 (M+H)⁺.

### Beispiel 2

In analoger Weise wie in Beispiel 1 beschrieben, wurden die folgenden Verbindungen hergestellt:
- Aus (RS)-2-Benzyl-3-[2-(2,2-dimethylpropionyloxy)-1,1-dimethyläthylsulfonyl]propionsäure und (S)-2-Amino-N-[(1S,2R,3S)-1-cyclohexylmethyl-3-cyclopropyl-2,3-dihydroxypropyl]-3-pyridin-3-ylpropionamid das weniger polare Epimer 2,2-Dimethylpropionsäure 2-[(R)- oder 2-[(S)2-[(S)-1-[(1S,2R,3S)-1-cyclohexylmethyl-3-cyclopropyl-2,3-dihydroxypropylcarbamoyl]-2-pyridin-3-yläthylcarbamoyl]-3-phenylpropylsulfonyl]-2-methylpropylester , MS : 742 (M+H)⁺, und das polarere Epimer 2,2-Dimethylpropionsäure 2-[(S)- oder 2[(R)-2[(S)-1-[(1S,2R,3S)-1-cyclohexylmethyl-3-cyclopropyl-2,3-dihydroxypropylcarbarmoyl]-2-pyridin-3-yläthylcarbamoyl]-3-phenylpropylsulfonyl]-2-methylpropylester, MS : 742 (M+H)⁺, jeweils als farbloser Schaum;
- aus (RS)-3-(2-Acetoxy-1,1-dimethyläthylsulfonyl)-2-benzylpropionsäure und (S)-2-Amino-N-[(1S,2R,3S)-1-cyclohexylmethyl-3-cyclopropyl-2,3-dihydroxypropyl]-3-imidazol-4-ylpropionamid das weniger polare Epimer Essigsäure 2-[(R)- oder 2-[(S)-2-[(S)-1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester , MS : 689 (M+H)⁺, und das polarere Epimer Essigsäure 2-[(S)- oder 2[(R)-2-[(S)-1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester , MS : 689 (M+H)⁺, jeweils als farbloser, amorpher Festkörper;
- aus (RS)-2-Benzyl-3-(2-hydroxy-1,1-dimethyläthylsulfonyl)propionsäure und (S)-2-Amino-N-[(1S,2R,3S)-1-cyclohexylmethyl-3-cyclopropyl-2,3-dihydroxypropyl]-3-imidazol-4-ylpropionamid das weniger polare Epimer (R oder S)-2-Benzyl-N-[(S)-1-[(1S,2R,3S)-1-cyclohexylmethyl-3-cyclopropyl-2,3-dihydroxypropylcarbamoyl]-2-imidazol-4-yläthyl]-3-(2-hydroxy-1,1-dimethyläthylsulfonyl)propionamid , MS : 647 (M+H)⁺, und das polarere Epimer (S oder R)-2-Benzyl-N-[(S)-1-[(1S,2R,3S)-1-cyclohexylmethyl-3-cyclopropyl-2,3-dihydroxypropylcarbamoyl]-2-imidazol-4-yläthyl]-3-(2-hydroxy-1,1-dimethyläthylsulfonyl)propionamid , MS : 647 (M+H)⁺, jeweils als amorpher, farbloser Festkörper.

Das als Ausgangsstoff eingesetzte (S)-2-Amino-N-[(1S,2R,3S)-1-cyclohexylmethyl-3-cyclopropyl-2,3-dihydroxypropyl]-3-pyridin-3-ylpropionamid wurde wie folgt hergestellt:
Zu einer Lösung von 878 mg (3.3 mMol) N-(tert-Butoxycarbonyl)-3-(pyridin-3-yl)-L-alanin (DE 3.640.535) und 750 mg (3.3 mMol) (1S,2R,3S)-3-Amino-4-cyclohexyl-1-cyclopropylbutan-1,2-diol in 33 ml Acetonitril wurden 1.27 g (3.3 mMol) HBTU und 0.45 ml (3.3 mMol) Triäthylamin gegeben und über Nacht bei Raumtemperatur gerührt. Die nach Filtration erhaltenen farblosen Kristalle löste man in 30 ml Äthanol und gab 23.3 ml 1N Salzsäure dazu. Nach 24 Stunden bei 50° wurde die Lösung mit 2N Natronlauge basisch gestellt, unter vermindertem Druck eingedampft, und der Rückstand zwischen Wasser und Essigester verteilt. Die wäßrige Phase wurde dreimal mit Essigester extrahiert, und die vereinigten organischen Phasen über Magnesiumsulfat getrocknet, filtriert und unter vermindertem Druck eingedampft. Die chromatographische Reinigung des Rückstandes (Kieselgel, Methylenchlorid/Methanol/ Ammoniak 140:10:1) lieferte 436 mg (S)-2-Amino-N-[(1S,2R,3S)-1-cyclohexylmethyl-3-cyclopropyl-2,3-dihydroxypropyl]-3-pyridin-3-ylpropionamid, MS: 267 (M+H)⁺, als farblosen Schaum.

Die als Ausgangsstoffe eingesetzten Dihydrozimtsäurederivate wurden wie folgt hergestellt:
(a) (RS)-3-(2-Acetoxy-1,1-dimethyläthylsulfonyl)-2-benzylpropionsäure:
   In analoger Weise wie in Beispiel 1 (g-h) beschrieben, wurde durch Acylierung von (RS)-2-Benzyl-3-(2-hydroxy-1,1-dimethyläthylsulfonyl)-propionsäurebenzylester mit Acetylchlorid in Pyridin der (RS)-3-(2-Acetoxy-1,1-dimethyläthylsulfonyl)-2-benzylpropionsäurebenzylester, MS : 341 (M-Benzyl)⁺, als farbloses Öl erhalten. Die anschließende katalytische Hydrierung lieferte die (RS)-3-(2-Acetoxy-1,1-dimethyläthylsulfonyl)-2-benzylpropionsäure, MS : 296 (M-HCOOH)⁺, als farbloses Öl.
(b) (RS)-2-Benzyl-3-(2-hydroxy-1,1-dimethyläthylsulfonyl)propionsäure:
   In analoger Weise wie in Beispiel 1 (h) beschrieben, wurde durch katalytische Hydrierung über Palladium/Kohle (5 %ig) des in Beispiel 1 (f) beschriebenen (RS)-2-Benzyl-3-(2-hydroxy-1,1-dimethyläthylsulfonyl)-propionsäurebenzylesters die (RS)-2-Benzyl-3-(2-hydroxy-1,1-dimethyläthylsulfonyl)propionsäure als farbloser Festkörper erhalten; MS : 300 (M)⁺.

### Beispiel 3

In analoger Weise wie in Beispiel 1 beschrieben, wurde durch Kondensation von (RS)-2-Benzyl-3-(1-benzyloxycarbonyl-1-methyläthylsulfonyl)propionsäure mit (S)-2-Amino-N-[(1S,2R,3S)-1-cyclohexylmethyl-3-cyclopropyl-2,3-dihydroxypropyl]-3-imidazol-4-ylpropionamid das weniger polare Epimer 2-[(S)- oder 2-[(R)-2-[(S)-1-[(1S,2R,3S)-1-Cyclohexylmethyl-3-cyclopropyl-2,3-dihydroxypropylcarbamoyl]-2-imidazol-4-yläthylcarbamoyl]-3-phenylpropylsulfonyl]-2-methylpropionsäurebenzylester , MS : 751 (M+H)⁺, und das polarere Epimer 2-[(R)- oder 2-[(S)-2-[(S)-1-[(1S,2R,3S)-1-Cyclohexylmethyl-3-cyclopropyl-2,3-dihydroxypropylcarbamoyl]-2-imidazol-4-yläthylcarbamoyl]-3-phenylpropylsulfonyl]-2-methylpropionsäur benzylester , MS : 751 (M+H)⁺, jeweils als farbloser Schaum erhalten.

Die als Aussgangsstoff eingesetzte (RS)-2-Benzyl-3-(1-benzyloxycarbonyl-1-methyläthylsulfonyl)propionsäure wurde wie folgt hergestellt:
(a) (RS)-2-(2-tert-Butoxycarbonyl-2-benzyläthylsulfonyl)-2-methylpropionsäure:
   In analoger Weise wie in Beispiel 1 (d) beschrieben, wurde durch Addition von 2-Mercaptoisobuttersäure an 2-Benzylacrylsäure-tert-butylester, hergestellt nach allgemein bekannter Verfahrensweise aus 2-Benzylacrylsäure und N,N-Dimethylformamid-di-tertbutylacetal [Tetrahedron 1979, 35, 1675)], die (RS)-2-(2-tert-Butoxycarbonyl-2-benzyläthylsulfonyl)-2-methylpropionsäure als gelbliches Öl erhalten; MS : 282 [M-H₂C=C(CH₃)₂]⁺.
(b) (RS)-2-(2-tert-Butoxycarbonyl-2-benzyläthylsulfonyl)-2-methylpropionsäur benzylester :
   Eine Lösung von 500 mg (1.3 mMol) (RS)-2-(2-tert-Butoxycarbonyl-2-benzyläthylsulfonyl)-2-methylpropionsäure in 5 ml Acetoritril wurde mit 197 mg (1.3 mMol) DBU versetzt, und dazu wurden bei Raumtemperatur 222 mg (1.3 mMol) Benzylbromid getropft. Die gelbliche Reaktionslösung wurde weitere 3 Stunden gerührt, dann mit 10 ml Wasser versetzt und zweimal mit je 10 ml Essigester extrahiert. Die vereinigten Essigesterphasen wurden zweimal mit je 5 ml Wasser gewaschen, anschließend über Natriumsulfat getrocknet und unter vermindertem Druck eingedampft. Der so erhaltene Benzylester wurde in Form eines gelben Öls (580 mg) ohne weitere Reinigung und Charakterisierung in die folgende Oxidationsstufe eingesetzt.
   Die Oxidation mit Kaliummonopersulfat-tripelsalz erfolgte analog Beispiel 1 (d) und ergab den (RS)-2-(2-tert-Butoxycarbonyl-2-benzyläthylsulfonyl)-2-methylpropionsäurebenzylester als farbloses Öl; MS : 461 (M+H)⁺.
(c) (RS)-2-Benzyl-3-(1-benzyloxycarbonyl-1-methyläthylsulfonyl)propionsäure :
   Ein Gemisch von 401 mg (0.87 mMol) (RS)-2-(2-tert-Butoxycarbonyl-2-benzyläthylsulfonyl)-2-methylpropionsäurebenzylester und 5 ml wasserfreier Ameisensäure wurde bei Raumtemperatur gerührt. Nach 6 Stunden war die anfangs trübe Lösung klar geworden. Diese wurde unter vermindertem Druck bei 40 ° eingedampft, und der Rückstand in 10 ml Esssigester aufgenommen und mit 3 ml Wasser gewaschen. Nach dem Trocknen über Natriumsulfat wurde im Wasserstrahlvakuum eingedampft, wobei 340 mg (RS)-2-Benzyl-3-(1-benzyloxycarbonyl-1-methyläthylsulfonyl)propionsäure , MS : 313 (M-Benzyl)⁺, als farbloses Öl erhalten wurden, welche ohne weitere Reinigung in die folgende Stufe eingesetzt wurde.

### Beispiel 4

In analoger Weise wie in Beispiel 1 (h) beschrieben, wurden durch katalytische Hydrierung die folgenden Verbindungen hergestellt:
- Aus 2-[(S)- oder 2-[(R)-2-[(S)-1-[(1S,2R,3S)-1-Cyclohexylmethyl-3-cyclopropyl-2,3-dihydroxypropylcarbamoyl]-2-imidazol-4-yläthylcarbamoyl]-3-phenylpropylsulfonyl]-2-methylpropionsäurebenzylester die 2-[(S)- oder 2-[(R)-2-[(S)-1-[(1S,2R,3S)-1-Cyclohexylmethyl-3-cyclopropyl-2,3-dihydroxypropylcarbamoyl]-2-imidazol-4-yläthylcarbamoyl]-3-phenylpropylsulfonyl]-2-methylpropionsäure als farbloser, amorpher Festkörper; MS : 661 (M+H)⁺, und
- aus 2-[(R)- oder 2-[(S)-2-[(S)-1-[(1S,2R,3S)-1-Cyclohexylmethyl-3-cyclopropyl-2,3-dihydroxypropylcarbamoyl]-2-imidazol-4-yläthylcarbamoyl]-3-phenylpropylsulfonyl]-2-methylpropionsäurebenzylester die 2-[(R)- oder 2-[(S)-2-[(S)-1-[(1S,2R,3S)-1-Cyclohexylmethyl-3-cyclopropyl-2,3-dihydroxypropyl carbamoyl]-2-imidazol-4-yläthylcarbamoyl]-3-phenylpropylsulfonyl]-2-methylpropionsäure als farbloser, amorpher Festkörper; MS : 661 (M+H)⁺.

### Beispiel A

### Orale, wässrige Suspension

| Zusammensetzung: | |
|---|---|
| 2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester, mikronisiert | 5.0 g |
| Polysorbat 80 | 0.3 g |
| Hydroxypropylmethylcellulose | 1.0 g |
| Geschmackstoff | q.s. |
| Methylparaben | 0.2 g |
| Propylparaben | 0.04 g |
| Wasser | ad 100.0 ml |

### Beispiel B

### Orale, wässrige Lösung

| Zusammensetzung: | |
|---|---|
| 2,2-Dimethylpropionsäure 2-[(S)- oder 2-[(R)-2-[(S)-1-[(1S,2R,3S)-1-cyclohexylmethyl-3-cyclopropyl-2,3-dihydropropylcarbamoyl]-2-imidazol-4-yläthylcarbamoyl]-3-phenylpropylsulfonyl]-2-methylpropylester, | 1.0 g |
| Methylparaben | 0.2 g |
| Propylparaben | 0.04 g |
| Geschmackstoff | q.s. |
| Wasser | ad 100.0 ml |

### Beispiel C

**Tabletten**

| Zusammensetzung: | | |
|---|---|---|
| 1) | 2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester | 200 mg |
| 2) | Milchzucker wasserfrei | 160 mg |
| 3) | Hydroxypropylmethylcellulose | 18 mg |
| 4) | Natrium-carboxymethylcellulose | 20 mg |
| 5) | Magnesiumstearat | 2 mg |
| | | Tablettengewicht 400 mg |

### Herstellungsverfahren:

1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt und zu Tabletten geeigneter Größe verpreßt.

### Beispiel D

### Kapseln

| Zusammensetzung: | | |
|---|---|---|
| 1) | 2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester | 200 mg |
| 2) | Milchzucker wasserfrei | 160 mg |
| 3) | Hydroxypropylmethylcellulose | 18 mg |
| 4) | Natrium-carboxymethylcellulose | 20 mg |
| 5) | Magnesiumstearat | 2 mg |
| | | Kapselfüllgewicht 400 mg |

### Herstellungsverfahren:

1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt, das Gemisch in Kapseln geeigneter Größe abgefüllt.

### Beispiel E

### Injektionslösung

| Zusammensetzung: | |
|---|---|
| | 1 ml |
| 2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-imidazol-4-yläthylcarbamoyl]-3-phenylpropylsulfonyl]-2-methylpropylester | 20 mg |
| D-Mannit pyrogenfrei | 10 mg |
| Wasser zu Injektionszwecken | ad 1.0 ml |

### Herstellungsverfahren:

Der Wirkstoff und das Mannit werden in Stickstoff-begastem Wasser gelöst und anschließend nach einem üblichen Verfahren lyophilisiert.

### Beispiel F

Wenn man nach den in den Beispielen A-E beschriebenen Verfahren arbeitet, können aus den folgenden, ebenfalls bevorzugten Verbindungen und ihren pharmazeutisch verwendbaren Salzen entsprechende galenische Präparate hergestellt werden:
2,2-Dimethylpropionsäure 2-[(R)- oder 2-[(S)-2-[(S)-1-[(1S,2R,3S)1-cyclohexylmethyl-3-cyclopropyl-2,3-dihydroxypropylcarbamoyl]-2-pyridin-3-yläthylcarbamoyl]-3-phenylpropylsulfonyl]-2-methylpropylester und
2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-pyridin-3-yläthylcarbamoyl]-3-phenylpropylsulfonyl]-2-methylpropylester.

## Patentansprüche

1. Aminosäurederivate der allgemeinen Formel 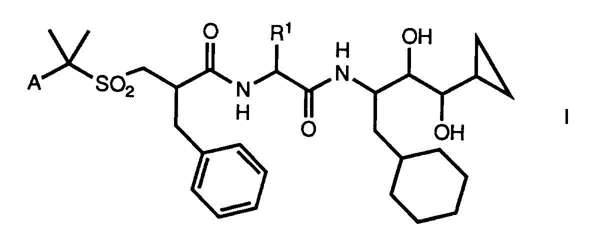 worin R¹ Imidazolylmethyl oder Pyridylmethyl und A Carboxyl, Benzyloxycarbonyl, Hydroxymethyl oder Alkylcarbonyloxymethyl bedeuten,
in Form von optisch reinen Diastereomeren, Diastereomerengemische, diastereomeren Racematen oder Gemischen von diastereomeren Racematen sowie pharmazeutisch verwendbare Salze dieser Verbindungen.

2. Verbindungen gemäss Anspruch 1, worin A Hydroxymethyl oder Alkylcabonyloxymethyl, besonders bevorzugt Alkylcarbonyloxymethyl, insbesondere C₁₋₄-Alkylcarbonyloxymethyl, bedeutet.

3. Verbindungen gemäss Anspruch 1, worin A Carboxyl bedeutet.

4. 2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-imidazol-4-yl-äthylcarbamoyl]-3-phenylpropylsulfonyl]-2-methylpropylester.

5. 2,2-Dimethylpropionsäure 2-[(R)- oder 2-[(S)-2-[(S)-1-[(1S,2R,3S)-1-cyclohexylmethyl-3-cyclopropyl-2,3-dihydroxypropylcarbamoyl]-2-pyridin-3-yläthylcarbamoyl]-3-phenylpropylsulfonyl]-2-methylpropylester.

6. 2,2-Dimethylpropionsäure 2-[(S)- oder 2-[(R)-2-[(S)-1-[(1S,2R,3S)-1-cyclohexylmethyl-3-cyclopropyl-2,3-dihydroxypropylcarbamoyl]-2-pyridin-3-yläthylcarbamoyl]-3-phenylpropylsulfonyl]-2-methylpropylester.

7. Verbindungen der allgemeinen Formeln 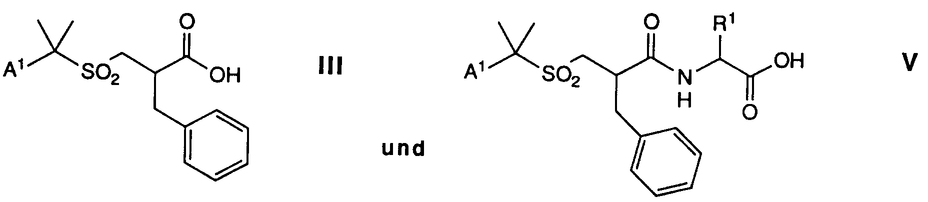 worin R¹ Imidazolylmethyl oder Pyridylmethyl und A¹ Benzyloxycarbonyl, Hydroxymethyl oder Alkylcarbonyloxymethyl bedeuten.

8. Aminosäurederivate gemäss einem der Ansprüche 1-6 zur Anwendung als therapeutische Wirkstoffe.

9. Aminosäurederivate gemäss einem der Ansprüche 1-6 zur Anwendung bei der Bekämpfung bzw. Verhütung von Bluthochdruck und Herzinsuffizienz.

10. Verfahren zur Herstellung einer Verbindung gemäss einem der Ansprüche 1-6, dadurch gekennzeichnet, dass man
a) eine Verbindung der allgemeinen Formel 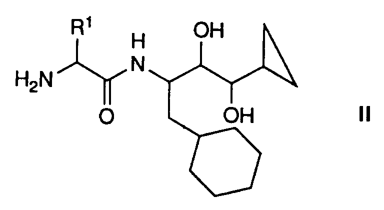 worin R¹ die in Anspruch 1 angegebene Bedeutung besitzt, mit einer Säure der allgemeinen Formel 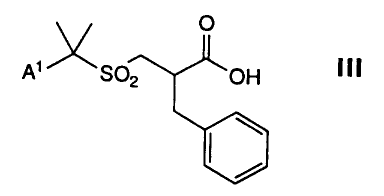 worin A¹ Benzyloxycarbonyl, Hydroxymethyl oder Alkylcarbonyloxymethyl bedeutet,
oder einem aktivierten Derivat davon umsetzt, oder
b) die Verbindung der Formel 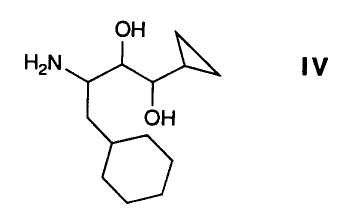 mit einer Säure der allgemeinen Formel 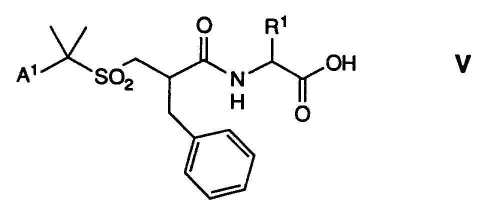 worin A¹ die oben und R¹ die in Anspruch 1 angegebene Bedeutung besitzen,
oder einem aktivierten Derivat davon umsetzt, oder
c) zur Herstellung einer Verbindung der Formel I, worin A Carboxyl bedeutet, in einer Verbindung der Formel I, worin A Benzyloxycarbonyl bedeutet, die Benzylgruppe abspaltet, und
d) erwünschtenfalls ein Gemisch diastereomerer Racemate in die diastereomeren Racemate oder optisch reinen Diastereomeren auftrennt, und/oder
e) erwünschtenfalls ein Diastereomerengemisch in die optisch reinen Diastereomeren auftrennt, und/oder
f) erwünschtenfalls eine erhaltene Verbindung in ein pharmazeutisch verwendbares Salz überführt.

11. Arzneimittel, enthaltend ein Aminosäurederivat gemäss einem der Ansprüche 1-6 und ein therapeutisch inertes Excipiens.

12. Mittel zur Bekämpfung bzw. Verhütung von Bluthochdruck und Herzinsuffizienz, enthaltend ein Aminosäurederivat gemäss einem der Ansprüche 1-6 und ein therapeutisch inertes Excipiens.

13. Verwendung eines Aminosäurederivates gemäss einem der Ansprüche 1-6 zur Herstellung von Mitteln gegen Bluthochdruck und/oder Herzinsuffizienz.
